# EUROPEAN PATENT APPLICATION

(11) **EP 3 970 683 A1**
(43) Date of publication of application: **23.03.2022**
(21) Application number: 20805636.6
(22) Date of filing: 31.01.2020
(51) Int. Cl.: A61H 3/00, A61B 5/11

(54) **MULTIFUNCTIONAL UNBLOCKING DEVICE**

(30) Priority: 14.05.2019 ES 201900245 U
(71) Applicant: I4Life Innovation y Desarrollos S.L., Potter 183 - 33203 Gijón (Asturias) (ES)
(72) Inventor: GARCíA PRIETO, Maria Antonia, 33203 Gijón (Asturias) (ES)
(74) Representative: Herrero & Asociados, S.L.
(86) International application number: PCT/ES2020/000006
(87) International publication number: WO 2020/229712

(57) **Abstract**

Multifunctional unblocking device consisting of a device designed to be incorporated into technical aids used by certain patients in order to overcome blocking episodes and improve their quality of life, characterized in that, when integrated in the handle of special walking sticks or carts or the like, it includes an ordinary laser beam that projects a straight segment on the ground, a complementary laser beam that provides a projection in the form of a spot, a vibrator with effect on the device itself and transmission by Bluetooth to a bracelet, worn by the user on the wrist or ankle, a mechanical or electronic gyroscope, a GPS pager, wireless connection means such as Wi-Fi and RFID (radiofrequency identification}, linking to a mobile phone with a guidance application and output by speaker or headphones, contactless transmission means (NFC), meters of speed, acceleration and vital signs of the user, radio receiver and music player, broadcast means for sending all user data to an internet site accessible both by the medical monitoring center and by the center for the monitoring by a relative or caregiver.

## Description

### Technical object of the invention

It consists of a device designed to be incorporated into walking sticks, walking frames, shopping cart or any other technical aid used by the patient which includes, on the one hand, a series of signals or stimuli that facilitate unblocking and paging, useful functionalities for the user herself/himself, on the other hand, modules for measuring various parameters and vital signs of the patient and finally, means of transmitting information that are sent to the doctor or caregiver who performs the monitoring so that all this results in an improvement of the quality of life of the user that is also permanently being paged.

### Technical sector to which the invention relates

The invention that is presented affects the Section of Current Needs of Life in its section of Health Protection Diversity, Paragraph Medical Sciences, Hygiene, influencing, from the industrial point of view, in the manufacture and commercialization of support devices for patients with episodes of motor freezing, disorientation and blockings of all kinds.

### Background of the Invention

Blocking episodes are very limiting events for those who suffer from them. To help patients in their resolution, what has been done, so far, is to instruct them in some very elementary techniques such as remembering a music so that they can walk at their own pace or throw an object on the ground, in front of their feet so that they try to step on it or imagine drawn lines that the patient can step on. Some patients have tapes stuck to the floor at home to avoid blockings. But these techniques have been proven to be not very effective.

For this reason there are some inventions such as the one described in US 2006025634 A1, wherein a shoe attached to a tab that hits the foot is described as a technique used to facilitate unblocking. Inventions US 6330888 81 and ES2362768 describe a walking stick at the base of which a tab appears so that the user steps on it to unlock. Invention US 2004014441 1 A1 describes a rod that is released so that the user steps on it and thus resumes its walk. The greatest drawback of the inventions described is the danger of drops that they imply.

Another invention, such as WO201592089 A1, which describes a shoe that prevents falls in Parkinson's disease patients, is an example that solves the problems associated with walking disorders.

Patent US 20060292533 A1 describes a device, housed in a walking frame, which provides lights and audible warnings to facilitate the walk recovery for the patient after a blocking episode.

In patents US 20060025836 A1 US5575294A solutions based on the emission of laser beams are described as an aid to unblocking.

Finally, perfecting the records just cited, mention should be made of Labor Invention ES1217449 U, with application number U 201800393, entitled "Luminous device to unblock walking connected with software that records its occurrence", which refers to a device that projects a light beam, in the area close to the patient's feet, activated automatically by a blocking detection module or by pressing a switch by the user himself, all that with means that register and even predict blocking with the added novelty of sending the information to a medical center for its knowledge, study and monitoring with the ultimate aim of helping the patient and deepening the analysis of these blocking phenomena.

In view of all this and with the experience acquired in the development of prototypes, an even better device has been conceived, which is the one described in this document, including the following novelties:
- complementary laser beam that provides a spot on the ground visible even in strong sunlight.
- gyroscope that cancels laser radiation when it is not pointed at the mechanical vibrating floor on the device itself with the option of duplicating it with an ankle or wrist bracelet
- indoor and outdoor paging device
- activity measurement and study of the user's walk
- basic aids such as telephone for emergencies, loudspeaker for guiding back home, radio, flashlight, payment in transport or commerce, etc.
- modules for measuring possible pathologies of the user (glucometer, heart rate monitor, oximeter, etc.
- linking to an application that uploads data to a cloud accessible to the doctor, family member or caregiver
- incorporation in a specific walking stick for the user, in any walking stick, in a walking frame, in a shopping cart or in any technical aid used by the patient.

All of these novelties entail significant improvements in the quality of life of the patient who suffers this type of blocking or disorientation episodes because it directly informs the doctor and/or caregivers of their occurrence, and similar devices are not known in the current state of the art.

### Summary description of the invention

The present invention, as noted above, refers to a device that includes a series of elements intended to facilitate, on the one hand, the unblocking of the motor system of certain patients who suffer occasional blocking episodes and, on the other hand, to provide security and confidence for this type of user when they are alone away from home or from their usual caregivers.

Conceived for incorporation into technical aids such as walking sticks, walking frames, carts or similar devices, it is small in size and has a series of elements that, in principle, can be classified as follows:
- those of autonomous activation by the user himself/herself
- those provided remotely
- those that transmit technical features related to the walk and vital signs of the patient
- those that provide distraction or security to the user.

The first ones are achieved with the use of laser beams combined or not with the generation of vibrations.

The second ones are based on linking to mobiles that allow transmitting instructions.

Third parties measure parameters of the user's walk and certain vital signs that are taken periodically with the issuance of results over the Internet so that they are known by doctors or caregivers.

The latter ones refer to being able to listen to radio stations, music or provide help of any kind.

All of them are designed to improve the quality of life of this type of patients with the advantage that, in addition to what they can do by themselves, they are monitored or supervised remotely to resolve possible emergencies and especially for the study of the various cases that provide greater knowledge in the medical field.

Schematic figures of the device and a complete description of the embodiments preferred by its inventor are included in the following sections.

### Brief description of the drawings

Five schematic figures are included, which are considered sufficient for the correct interpretation of the invention.
Figure 1
   It shows the case of the device of the invention incorporated, during the manufacturing process, in the handle of a walking stick specially designed for it.
   1.- Unblocking device on the handle of the walking stick
   2.- Cover
   3.- Sequential button
   4.- Shaft
Figure 2
   It shows a second embodiment of the unblocking device.
   1.1.- Unblocking device in sliding module on the handle of the walking stick.
Figure 3
   It shows a third embodiment of the unblocking device.
   1.2.- Unblocking device in sliding module on the shaft of the walking stick.
   5.- Grip
Figure 4
   A block diagram is represented that synthesizes the general configuration of the device.
   6.- Block A related to laser emission and vibration
   7.- Block B related to Gyroscope
   8.- Block C related to paging
   9.- Block D related to support means in emergencies
   10.- Block E related to walk activity parameters
   11.- Block F related to vital signs of the user
   12.- Block G related to battery and charging
   13.- Block H related to NFC connectivity
   14.1 - Medical monitoring center
   14.2 - Center for the monitoring by a relative or caregiver
   15.- File cloud
Figure 5
   A user is represented with technical assistance like a walking stick.
   16.- Ordinary laser
   16.1.- Straight segment projected by the ordinary laser
   17.- Complementary laser
   17.1- Spot projected by the complementary laser
   18.- Bracelet
   19.- User

### Detailed explanation of embodiments of the invention

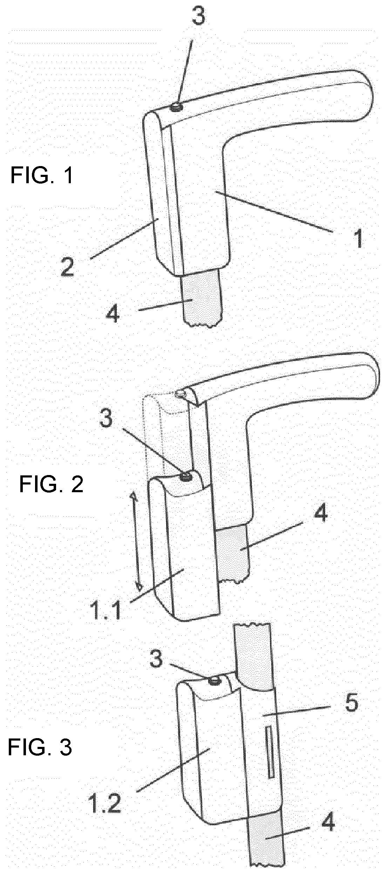

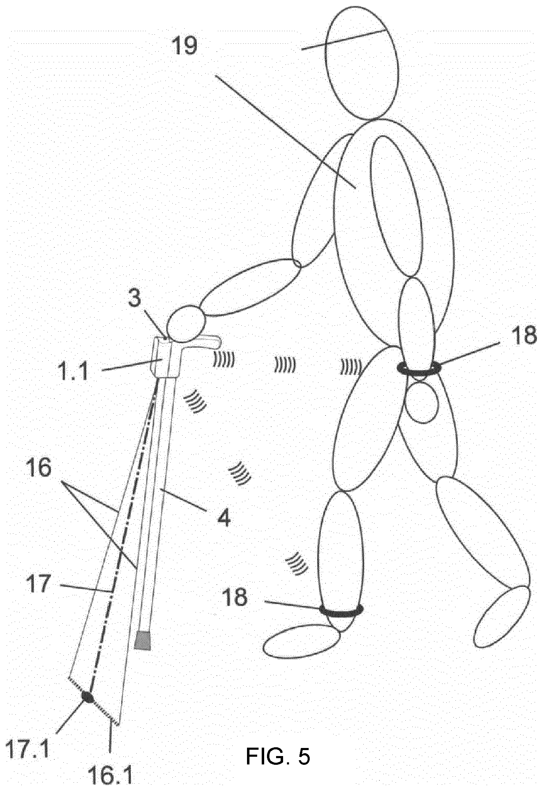

Multifunctional unblocking device (1) (Figs. 1 to 5) designed to be incorporated into technical aids used by certain patients in order to overcome blocking and disorientation episodes that, in general, improve their quality of life. In one embodiment preferred by its inventor, it is shown as a modular device that, in a first embodiment, represented in (Fig. 1), is integrated into the hollow handle of a usual walking stick, specially conceived for it, being practicable because it is equipped with a cover (2). In the upper external part of the device, in a place that is well accessible to the user, a sequential button (3) is installed that, in a first press, activates an ordinary laser beam (18) (Fig. 5), which projects a straight segment (16.1) on the ground, in a position in front of the user, so that the user can see it clearly as soon as the signal is activated. In a second press, the device emits a complementary laser beam (17) that is projected approximately in the central part of the straight segment (16.1), in the form of a spot (17.1), very visible even on days with high solar luminosity.

A third actuation on the sequential button (3), which complements the light-type excitations, causes a vibration of the device unlocked in the handle of the walking stick and/or a vibration in a bracelet (18) that the user wears on the wrist or ankle, when leaving home or when deemed appropriate. The aforementioned bracelet (18) is linked to the device via Bluetooth.

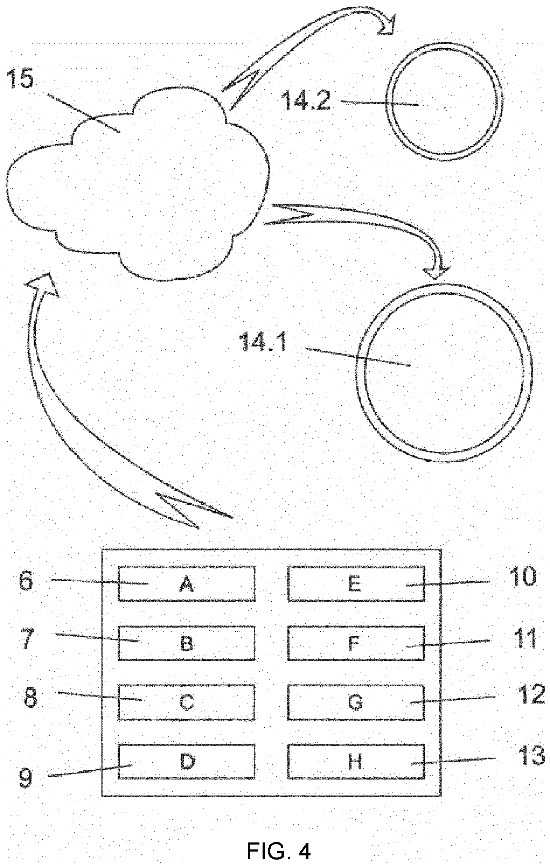

Both light signals and the vibratory effect, which are timed so that their extinction occurs after a predetermined time has elapsed, constitute a part of the general equipment of the device whose content is indicated, in the block diagram of (Fig. 4) corresponding said signals and effect to block A related to laser emission and vibration (6). In addition, the light emissions are also extinguished, automatically, by the action of what is contained in the block B related to the gyroscope (7), which responds to a classical mechanical gyroscope with three orthogonal axes or its equivalent in electronic embodiment. Its purpose is to avoid damage to the eyes of other people nearby or even the user himself/herself when, for any reason, the laser beams are not directed towards the ground.

The sequential button (3) can be replaced by means of automatic activation or by any other walk/stop technical means known and used in the industry.

These two blocks include elements of automatic operation or autonomous use by the user, that is, that the user activates them when he/she deems it necessary without prejudice to the fact that those in charge of monitoring them know everything that happens in real time or by accessing the files that are generated and stored in the cloud (15) of Internet.

Block C related to paging (8) incorporates various technologies. Using GPS, it allows the position of the device (both indoors and outdoors) to be known exactly at any time so that the user's caregivers can come to her/his aid and assist her/him in an emergency, which results in greater peace of mind for her/him and those who take care of her/him. It also incorporates the means of wireless connection of Wi-Fi technology, Bluetooth and radio frequency identification (RFID), for data transmission and reception. The functions of block D (9) have a very important purpose by including a browser, with the appropriate application that, via speakerphone or headphone output, gives instructions to return home in possible episodes of disorientation, even allowing the device to be paired with the user's mobile phone (19) without ruling out other auxiliary functions such as radio, music, flashlight, etc.

In block E related to walk activity parameters (10), elements are installed that measure parameters of the user's mobility, such as activity, speed or sudden accelerations and decelerations due to stumbles or falls.

In block F related to user's vital signs (11), meters of vital parameters such as heart rate, oxygen saturation, sugar level and other similar characteristics are installed.

These two blocks E and F refer to records of occasional or regularly scheduled events so that the doctor, family members or caregivers, in general, can know, analyze and even predict possible behaviors in specific situations. To do this, the device has broadcast means that permanently send information to the cloud (15) so that the medical monitoring center (14.1) or center for the monitoring by the family or caregiver (14.2) can access the files and proceed to carry out the pertinent studies.

In block G related to battery and charging (12), a rechargeable battery and a device for that purpose to enable it to be charged.

Lastly, block H related to NFC (contactless communication) connectivity (13) allows the user to benefit from the functionalities for payments in transport or in commerce.

It is, in short, a comprehensive device to help users who, due to their temporary or permanent impairments, require means of support in the course of their daily life.

### Other embodiments

In a second embodiment shown in (Fig. 2), the multifunctional unblocking device is a sliding module (1.1) installed in the handle of the walking stick.

In a third embodiment that is indicated in (Fig. 3), the multifunctional unblocking device, also of the sliding type (1.2), is installed on a grip (5) coupled to the shaft (4). It is understood that the device of the invention could also be attached to a walking frame, a shopping cart or any similar device.

It is not considered necessary to make the content of this description more extensive so that an expert in the field can understand the scope and advantages derived from the Invention, as well as develop and put into practice the object thereof. However, it should be understood that the invention has been described according to preferred embodiments thereof, so that it may be susceptible to modifications without affecting or implying any alteration of the basis of said invention. That is to say, the terms in which these embodiments of the invention have been set forth should always be taken on a broad and not limitative basis.

## Claims

1. Multifunctional unblocking device consisting of a device designed to be incorporated into technical aids used by certain patients in order to overcome blocking episodes and improve their quality of life, **characterized in that**, when integrated into the handle of special walking sticks or as a module to be attached to any walking stick or technical aid of the walking frame, cart or similar type, it includes an ordinary laser beam (16) that projects a straight segment (16.1) on the ground, a complementary laser beam (17) that provides a projection in the form of a spot (17.1), a vibrator with effect on the device itself and Bluetooth transmission to a bracelet (18), worn by the user on the wrist or ankle, a mechanical or electronic gyroscope, a GPS pager, wireless connection means such as WIFI and RFID (radio frequency identification), mobile phone link with guidance application and output by speaker or headphones, contactless transmission media (NFC), meters of speed, acceleration and vital signs of the user, radio receiver and music player, broadcast means for sending all user data (19) to an internet site accessible both by the medical monitoring center (14.1) and the center for the monitoring by a relative or caregiver (14.2).

2. Multifunctional unblocking device, according to claim one, **characterized in that** the activation of the laser beams and vibrator is carried out by means of a sequential button (3), by means of automatic activation or any other walk/stop technical means.

3. Multifunctional unblocking device, according to claim one, **characterized in that** the active periods of the ordinary laser beam (16), the complementary laser beam (17) and the vibrator are timed.

4. Multifunctional unblocking device, according to claim one, **characterized in that** it is powered by rechargeable batteries.
